# EUROPEAN PATENT SPECIFICATION

(11) **EP 2 067 027 B1**
(45) Date of publication and mention of the grant of the patent: **08.08.2012**
(21) Application number: 07825225.1
(22) Date of filing: 27.09.2007
(51) Int. Cl.: G01N 27/327, C12Q 1/00

(54) **MICROSENSOR FOR DETECTION OF D-AMINO-ACIDS**
MIKROSENSOR ZUM NACHWEIS VON D-AMINOSÄUREN
MICROCAPTEUR POUR LA DETECTION D'ACIDES AMINES D

(30) Priority: 27.09.2006 EP 06291523
(43) Date of publication of application: 10.06.2009
(73) Proprietor: CENTRE NATIONAL DE LA RECHERCHE SCIENTIFIQUE (CNRS), 75794 Paris Cedex 16 (FR); Universite Claude Bernard de Lyon 1, 69622 Villeurbanne Cedex (FR); Université Paris XI, 91405 Orsay Cedex (FR); Université d'Insubria, 21100 Varese (IT); Institute Of Molecular Biology And Genetics National Academy of Sciences of Ukraine, 04143 Kiev (UA)
(72) Inventor: MARINESCO, Stéphane, 69008 Lyon (FR); PERNOT, Pierre, 34090 Montpellier (FR); MOTHET, Jean-Pierre, 75020 Paris (FR); CESPUGLIO, Raymond, 69390 Millery (FR); SCUVAILO, Oleg, 69009 Lyon (FR); SOLDATKIN, Alexey, 04211 Kiev (UA); POLLEGIONI, Loredano, 20152 Milano (IT); PILONE, Mirella, 20121 Milano (IT)
(74) Representative: Novagraaf Technologies
(86) International application number: PCT/IB2007/002864
(87) International publication number: WO 2008/038129

(56) References cited:
- EP-A- 1 542 017
- WO-A-02/082970
- US-A1- 2006 079 740
- JOHANSSON E ET AL: "STUDY OF A REAGENT- NAD MEDIATORLESS BIOSENSOR FOR D-AMINO ACIDS BASED ON CO-IMMOBILIZED D-AMINO ACID OXIDASE AND PEROXIDASE IN CARBON PASTE ELECTRODES" JOURNAL OF BIOMATERIALS APPLICATIONS, TECHNOMIC, LANCASTER, PA, US, vol. 8, October 1993 (1993-10), pages 146-173, XP008075336 ISSN: 0885-3282
- QIJIN CHI ET AL: "AMPEROMETRIC BIOSENSORS BASED ON THE IMMOBILIZATION OF OXIDASES IN A PRUSSIAN BLUE FILM BY ELECTROCHEMICAL CODEPOSITION" ANALYTICA CHIMICA ACTA, ELSEVIER, AMSTERDAM, NL, vol. 310, 1995, pages 429-436, XP008075382 ISSN: 0003-2670

## Description

The instant invention relates to the field of microsensors or sensing microdevices.

More precisely the invention concerns a microsensor or microelectrode for measurement of D-serine and an electrochemical method for detecting and/or measuring D-amino acid, in particular D-serine, more specifically *in vitro, ex vivo* and/or *in vivo.*

There is specially a need for D-amino-acid sensors that can be used for *in vivo* measurement. For example, for D-serine, which has been recently shown to be present in the Central Nervous System (CNS), in the cortex, hippocampus or developing cerebellum.

This D-amino acid has been recently implicated in several pathologies such as schizophrenia, Alzheimer disease, chronic pain or cerebral ischemia. There is thus a need for pharmacological agents able to interfere with synthesis, release, catabolism and/or uptake system of D-serine in the CNS, as well as for reliable methods for detecting D-serine *in vivo* and *in vitro*.

A known method to measure extracellular concentration of D-serine is microdialysis. This method is heavy, expensive and difficult to employ. Moreover, it involves the use of relatively large probes, which may provoke lesions that can compromise the measurement.

Furthermore the principle of this method by itself, comprising the steps of dialysing the extracellular medium, collecting and analysing its content, can induce a perturbation in the physiological functionality of the sample due to the circulation of exogenous extracellular fluid, which may change the local concentration of D-serine as well as of other small metabolites.

Electrochemical methods to detect D-serine are also known, for example in food industry. These methods use sensors having a millimetric or centimetric size, which is generally not compatible with an *in vivo* use.

The field of microsensors is of increasing interest. There is thus a general need for reliable, cheap, small, precise, selective and/or versatile sensors and more particularly for sensors which can be used *in vivo* and/or allowing to measure in real time the changes in the concentration of a compound.

EP1542017 relates to a method of examining or diagnosis schizophrenia by measuring concentration of D-Serine by chromatography.

Johanssonn et al. (J. of Biomaterials Application, vol 8, p. 146-173, 1993) discloses a biosensor constituted by a 1.7mm diameter electrode modified with D-amino acid oxydase and either horse-radish peroxidase or fungal peroxidase.

Qijin Chi et al (Analytica Chimica Acta, vol 310, p. 429-436, 1995) discloses amperometric biosensor by incorporation of D-amino oxidase into a Prussian blue film during the electrochemical growth process and electrochemical deposition onto a 7 mm diameter basal graphite microelectrode.

As discussed above, there is a special need for a device for detecting D-amino-acids, and in particular D-serine, *in vivo* and/or allowing to measure in real time its concentration, for example, in order to develop a method to find pharmacological agents able to interfere with synthesis, release and/or elimination of D-amino-acids, and more specifically D-serine in the central nervous system (CNS).

Following a first aspect, the subject matter of the invention is a microelectrode, in particular for measuring the concentration of a D-amino acids, said microelectrode comprising:
- means for oxidation of said D-amino acids into at least a compound B,
- means for optimising the detection of said compound B, and
- means for reducing interferences, in particular interferences due to the oxidation of other species than compound B.

The microelectrode of the invention may have a detection limit of 300 nM or less of the D-amino-acid of interest, in particular D-serine.

The microelectrode presents a good selectivity. For example, solutions of serotonin, dopamine, L-serine and glycine at 10 µM (concentrations much higher than the physiological concentrations of these molecules), do not generate signals bigger than 5% of the ones detected in the same concentration of D-serine.
Figure 1 shows an example experiment where 200 nM of hydrogen peroxide was injected in the recording chamber. The injection produces a step in the oxidation current at the working electrode.
Figure 2 shows the calibration of the microelectrode of the invention in increasing concentrations of hydrogen peroxide. The microelectrode's response is linear with concentration between 10 nM and at least 10 µM of hydrogen peroxide.
Figure 3 A and B shows the detection of D-serine using the microelectrode of the present invention.

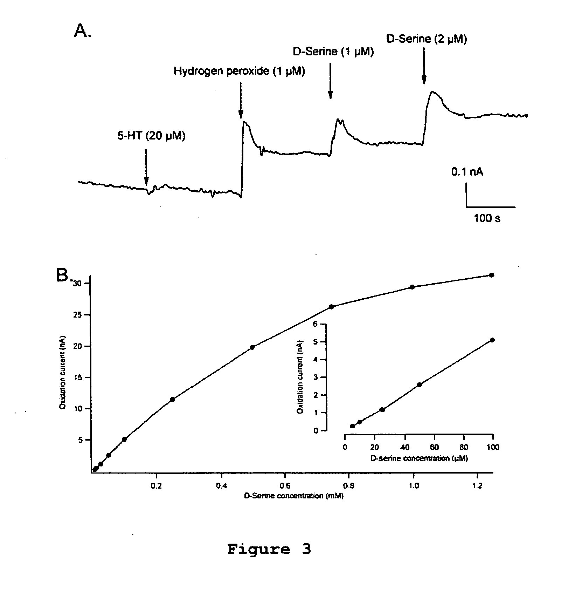

The Figure 3 A shows the recording oxidation (nA) current at microelectrode of the invention upon time (s) and application of serotonin (5-HT, 20 pM), hydrogen peroxide (H₂O₂, 1 µM), and D-serine (1 µM and 2 µM). D-serine and H₂O₂ are detected as a step in oxidation current, and interference from 5-HT is rejected.

The Figure 3 B shows the calibration curve of a microelectrode of the present invention in D-serine concentrations ranging from 5 µM to 1.2 mM.

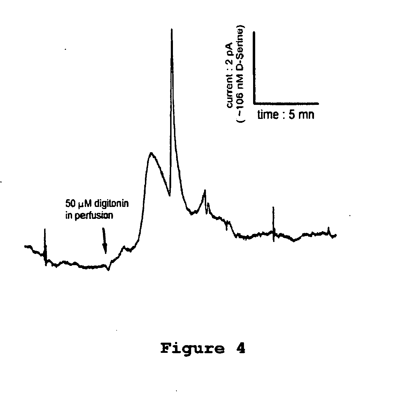

Figure 4 shows an example of release of D-serine by astrocytes in culture after application of digitonin. Digitonin 50 µM was added to cells in culture. The microelectrode of the present invention, placed above the cells detects the release of D-serine due to the disruption of the astrocytes cellular membrane.

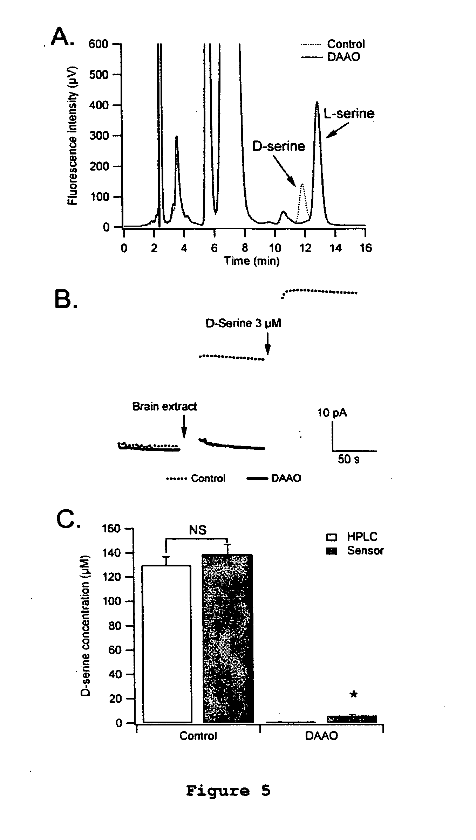

Figure 5 A, B and C show the excellent selectivity of the microelectorde of the present invention.

Figure 5 A is a typical chromatogram showing a curve of fluorescence intensity (µV) versus time (min). It shows an example D-serine peak after 12 min elution time and its disappearance after incubation, with *Rhodotorula gracilis* D-Amino Acid Oxidase (RgDAAO) and catalase.

The Figure 5 B shows an example of current response recorded at the microelectrode of the present invention upon addition of a brain extract (40X dilution) and 3 µM D-serine (oxidation current in pA versus time in s). The current response to a brain extract preincubated in RgDAAO and catalase was greatly reduced compared to that of a control brain extract. The Figure 5 C is a diagram showing summary results of the comparison between D-serine concentration estimated by HPLC and the microelectrode of the present invention.

By "microelectrode" in the present invention is meant a small size electrode, in particular an electrode having a mean diameter of less than 1 mm, specially of less than 500 µm, more particularly of less than 250 µm, especially of less than 150 µm and more specifically less than 100 µm, or even less than 50 µm.

The definition of a "microelectrode" according to the International Union of Pure and Applied Chemistry is as follows: "microelectrode is any electrode whose characteristic dimension is, under given experimental conditions, comparable or smaller than the diffusion layer thickness." Stulik K, Amatore C, Holub K, Marecek V and Kutner W (2000) Microelectrodes. Definitions, characterization and applications (technical report). Pure Appl. Chem. 72: 1483-92. [1]

The means for oxidation of the D-amino acids may be at least one D-amino-acid oxidase (DAAO), in particular a D-amino-acid oxidase which leads to the production of hydrogen peroxide as compound B.

A D-amino-Acid Oxidase (DAAO) may be flavoenzyme that contains a molecule of covalently or non-covalently bound flavin adenine dinucleotide as cofactor, which is the site of redox reaction

More precisely, the means for oxidation of the D-amino acid may be at least one D-amino-acid oxidase (DAAO) which doesn't need the addition of a cofactor in the medium to oxidise the D-amino-acids. The DAAO may have a natural or artificial cofactor that is tightly bound to the apoprotein moiety (i.e., no inactive apoprotein is produced under the assay conditions), and/or a high specific activity.

The D-Amino-Acid Oxidase may have a high specific activity, which can be of at least 20, in particular at least 40, or even at least 55 units/mg for at least one, or only one, specific D-amino acids. More precisely, the D-Amino-Acid Oxidase may have a high specific activity, which can be of at least 20, in particular at least 40, or even at least 55 units/mg D-serine and/or least 50, in particular at least 75, or even at least 100 units/mg for D-alanine. One DAAO unit is defined as the amount of enzyme that converts 1 µmole of D-alanine per minute at 25 °C..

The means for oxidation of the D-amino-acid is a DAAO from a yeast or a microorganism. The DAAO may be chosen from the group comprising *Rhodotorula gracilis DAAO (RgDAAO), Trigonopsis variabilis DAAO, V. luteoalbum DAAO, F*. *oxysporum DAAO* and derivatives thereof.

By "derivatives" is meant in the instant invention, an enzyme having at least 65 %, in particular at least 75 %, more particularly at least 85 %, notably at least 90 %, even at least 95 %, even more particularly at least 99 % identity of the amino acids with the corresponding enzyme. The derivatives have the catalitic activity, for example a D-amino acid oxidase, in particular with an efficiency of the same order, i.e. at least 50 %, more specially at least 75 %, in particular at least 100 %, even more than 150 % of the efficiency of the original enzyme. The efficiency of the enzyme may be defined as the ratio K_{cat}/Kₘ, assayed with an oxygen electrode at pH 8.5 and 25 °C, at air oxygen saturation ([O₂] = 0.253 mM) [G. Molla, C. Vegezzi, M.S. Pilone, L. Pollegioni, Overexpression in Escherichia coli of a recombinant chimeric Rhodotorula gracilis D-amino acid oxidase, Prot. Express. Purif., 14 (1998) 289-294]. [2]

The D-amino acids for which the concentration is measured is a substrate of the D-Amino-Acid Oxidase (DAAO). The D-amino acids may be a natural or a synthetic D-amino acid. It may be an alpha, beta, gamma or omega D-amino acid.

The amino acids may be selected from the group of D-alanine, D-valine, D-leucine, D-isoleucine, D-methionine, D-proline, D-phenylalanine, D-tryptophan, D-serine, D-threonine, D-tyrosine, D-cysteine, D-asparagine, D-glutamine, D-lysine, D-arginine and D-histidine, in particular D-serine, and their derivatives.

The means for oxidation of the D-amino-acids may be immobilized on the electrode by:
- Covalent immobilization, for example
   b y using bifunctional agents, such as glutaraldehyde:
      a) by using vapors of the bifunctional agent, or
      b) by preliminary mixing solution of bifunctional agents with enzymatic mixture
- Entrapping D-Amino-Acid Oxidase (DAAO), for example into a reticular matrix, which can be formed by different types of polymeric reactions, in particular stimulated by UV, irradiation or chemical catalyst, or electrogenerated, like polymers of phenylenediamine, pyrrole or aniline,
- Immobilisation into the body of the electrode, such as paste type electrode, for example mix of enzyme, graphite powder, mediator, and/or
- Adsorption, in particular on a high surface layered material, such as cellulose acetate, nylon, inorganic gels (for example silica gel) and such types of membranes.

The means for optimising the detection of compound B may correspond to means for the catalysis of hydrogen peroxide oxidation.

The means for optimising the detection of said compound B may be selected from:
- an electrochemical pre-treatment of the electrode, in particular carbon based electrode, and
- deposition, for example via electrochemical or vacuum deposition, of one or several metals, such as metals catalysing the oxidation of compound B,
- deposition of an artificial system catalysing the oxidation of compound B, such as artificial peroxidase system, in particular based on Prussian Blue,
- Horseradish peroxidase (HRP), and
- use of nanoparticles, for example of Gold, Platinum, Silicon oxides, Zinc, carbon nanotubes, etc. In other words, the means for optimising the detection of said compound B may be selected from:
- an electrochemical pre-treatment of the electrode,
- deposition of at least one metal
- deposition of an artificial system catalysing the oxidation of compound B,
- Horse Radish peroxidase (HRP),
- use of nanoparticles, and
- a combination thereof.

When the means for optimising the detection of said compound B is a metal it can be selected from the metals comprising platinum, gold, ruthenium, rhodium, palladium, iridium, osmium, iron, chromium, nickel and tungsten.

The means for reducing interferences may be limiting oxidation of compounds other than compound B which may be oxidised by the electrode, in particular by limiting the access and/or the contact of these compounds with the electrode, in other words in particular by limiting the access and/or the contact of said compound other than the compound B with the electrode.

The means for reducing interferences may be selected from the group comprising:
- electrogenerated polymeric membranes, for example based on monomers like benzene and its derivatives, such as phenylenediamine, resorcinol, phenol; naphthalene and its derivatives; pyrrole and its derivatives; aniline and its derivatives; and combinations thereof,
- membranes deposited from solutions of polymers, charged, like Nafion, or neutral, like polyurethane and polyvinyl chloride, or derivatives of cellulose,
- non polymeric charged molecule layer, for example such a layer is deposited via solution, such as phospholipids and fatty acids, like stearic acid,
- use of at least an enzyme that degrades interfering molecule(s), for example ascorbic acid oxidase, in particular this enzyme may be immobilized on the electrode, and
- use of electrochemical methods allowing to discriminate between different molecules, for example pulsed amperometry, cyclic voltammetry or pulsed voltammetry.

The means for reducing interferences may be a layer of a compound selected from the group comprising, polymeta-, para- or ortho- phenylenediamine (PPD), substituted naphthalene-based polymers, rejecting membrane or a rejecting polymer membrane.

Among the electrode materials that can be used the following may be cited:
- electrodes based on platinum, rhodium, palladium, gold, ruthenium, possibly under an alloy form, for example with gold, iridium or other noble metals, among such alloys an example is platinum 90 % and iridium 10 %
- electrodes based on carbon materials, graphite (rods), glassy carbon (rods), carbon fibres, diamond,
- electrodes covered by nanoparticles of noble metals, carbon materials or a mixture thereof, and
- ceramic based amperometric electrodes.

Some type of electrode materials may be excluded as they possibly lead to inactivation of the D-Amino-Acid Oxidase (DAAO), in particular Ag and Hg electrodes.

The electrode shape may be a disk, a ring, a rod, a cylinder, a cone or a hemisphere.

Following a specific embodiment, the microelectrode comprises, or consists of, a carbon fibre with the working part covered by a layer of ruthenium, a layer of PPD and a layer of DAAO.

Following another aspect, the subject matter of the invention is a device for detecting and/or measuring the concentration of a D-amino-acid in a medium, in particular *in vivo*, comprising an electrode as defined above.

This device may further comprise an acquisition card driving an amplifier, in particular equipped with a two or three electrodes potentiostat.

This device may also comprise a working electrode and a reference electrode for the two-electrode system, and an additional auxiliary or counter electrode for the three-electrode system.

Following another aspect, the subject matter of the invention is a method for detecting and/or measuring the concentration of a D-amino-acid in a medium, in particular *in vivo*, comprising the following steps:
- a working microelectrode of the present invention is placed in said medium,
- a working potential is applied, in particular by cyclic voltammetry, pulsed voltammetry/amperometry or by applying a constant potential.

The potential may be fixed between -1 and +1 V vs. Ag/AgCl for the detection of peroxide (H₂O₂).

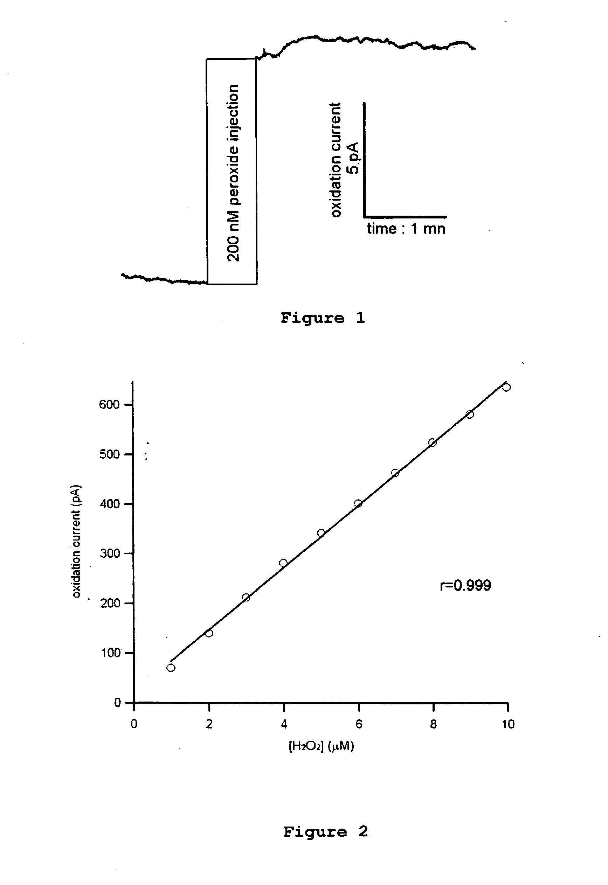

An efficient detection method is the continuous amperometry at a fixed potential of +0,55 V vs. Ag/AgCl. In this case the peroxide oxidation is seen via a jump of current at the level of working electrode (Fig. 1). The relationship between oxidation and peroxide concentration in the solution is linear, in particular in a range of 0,01 µM to 10 µM (Fig. 2).

Following another aspect, a subject matter of the invention is a method for making a microelectrode comprising the following steps:
- treating the electrode in order to increase its sensitivity to compound B, in particular via a metallization, for example an electrodeposition of a metal,
- the deposition of a polymer film in order to discriminate compound B from other compounds, i.e. other compounds present in the medium and, which may be oxidised by the electrode,
- the deposition of a film of an enzyme oxidising D-amino-acid to compound B on the electrode, and then immobilizing said enzyme, in particular via glutaraldehyde (vapours or solution).

The enzyme may be immobilised via a cross-linking process, for example with an aldehyde, such as glutaraldehyde, or with other molecules such as poly(ethylene glycol) 400 diglycidyl ester (PEGDGE) or may be imprisoned in the material of the electrode, such as the carbon paste. It can also be entrapped or encapsulated in a polymer film, such as polypyrrole.

The metal covering the working part of the electrode may be deposited via classical methods known from one skilled in the art, for example by a gel impregnated with the metal, for example the osmium hydrogel.

### Examples

### Example 1: Preparation of a ruthenium-coated carbon fibre microelectrode for measuring D-serine concentration

### 1. Preparation of the carbon fibre electrode

A 7 µm diameter carbon fibre electrode (Goodfellow Cambridge Ltd., Huntington, UK) glued to a copper wire by a silver paint (Radiospares, Beauvais, FR) deposition. The wire is placed in a glass micropipette on which the extremity is cut in order to allow the carbon fibre to protrude. The junction between glass micropipette and the carbon fibre is sealed by an epoxy resin, in order to insure its tightness, and the carbon fibre is cut at 150 µm.

The electrode is then put into ethanol for 20 minutes to clean the fibre of its impurities.

### 2. Electrodeposition of ruthenium

The electrodeposition of ruthenium has been done by applying for 20 minutes a potential of -450 mV Vs Ag/AgCl to the carbon fibre electrode which is in a solution of RuCl₃ at a concentration of 100 µg/ml and pH 2.5 (ruthenium atomic absorption standard solution, Sigma, Saint Quentin, France).

After the deposition of Ruthenium, the electrode has been cycled 15 times between 0 and 700 mV for stabilisation.

### 3. Deposition of a PPD film layer

The deposition has been done electrochemically by putting the electrode 20 minutes in a solution of 100 mM of m-PD and applying a potential of +700 mV vs. Ag/AgCl.

### 4. Deposition of a D-amino-acid oxidase (DAAO) film

A film of enzyme is deposited on the electrode by dipping the electrode into an enzyme solution comprising 56 mg/ml of RgDAAO, 25 mg/ml Bovine Serum Albumine (BSA) and 1 % glycerol. The electrode is removed from the solution and then the enzyme is immobilised by exposition to vapours of a 50 % glutaraldehyde solution.

Alternatively the enzyme may be immobilized by using a solution of enzyme comprising glutaraldehyde 0.17 % ; glycerol 2 % ; RgDAAO 37 mg/ml ; BSA 17 mg/ml.

The thickness of the DAAO film obtained is about 15 to 25 µm.

### Example 2: Preparation of a platinum disk-shaped electrode covered with yeast D-amino acid oxidase for D-serine detection.

Electrodes were prepared by inserting a 400 µm diameter platinum wire into a glass capillary and sealing the tip of the pipette by melting the glass over a flame. The platinum wire inside the pipette was then soldered to a copper wire using Pb-Sn solder and the back end of the pipette was sealed using epoxy resin. The tip of the electrode was then polished using sand paper of increasing fineness (finishing with 0.3 µm Al₂O₃ emery paste) in order to expose a clean 400 µm diameter Pt disk.

A first layer of poly-m-phenylenediamine was deposited by electropolymerization of m-phenylenediamine (see example 1). The enzymatic layer was deposited by laying a small drop (∼ 30 nl) of a solution of Rhodotorula Gracilis D-amino Acid Oxidase RgDAAO (56 mg/ml of RgDAAO, 25' mg/ml Bovine Serum Albumine (BSA) and 1 % glycerol) onto the Pt disk and exposing it to saturated glutaraldehyde vapors for 5 min. The electrode was then rinsed in Phosphate Buffer Saline (PBS), dried and stored at 4°C in dry atmosphere. The thickness of the resulting membrane was about 5-10 µm.

### Example 3: D-serine detection using the microelectrode of the present invention

The microelectrode used in this example was the same as in example 2.

A calibration curve in D-serine ranging from 5 µM to 1.2mM was done with the microelectrode of the example 2 (Figure 3 B). The microelectrode's response is linear with concentration between 5 µM and at least 100 µM.

The oxidation current at the microelectrode upon application of serotonin (5-HT, 20 µM), hydrogen peroxide (H₂O₂, 1 µM) , and D-serine (1 µM and 2 µM) was measured (Figure 3 A). D-serine and H₂O₂ were detected as a step in oxidation current, and interference from 5-HT was rejected (Figure 3B).

### Example 4: Devices for measuring the concentration of D-serine

The system of command and acquisition is composed of an acquisition card ITC-18 (Instrutech Corporation, Greatneck, NY, USA), driving an amplifier VA-10 (NPI Electronics, Tamm, Germany) equipped with a two-electrode (working electrode corresponding to the microelectrode of the invention and home made reference electrode composed of a chlorided silver wire) potentiostat. The acquisition card is driven with the SVoltare software.

### Example 5: cell cultures.

Primary cultures of cortical astrocytes were prepared from newborn rats and cultured until reaching confluence (14 days). The astrocytes were then treated 5 days with 8 µM cytosine arabinofuranoside in order to eliminate remaining microglia.

The cells were placed in a chamber perfused with Krebs buffer. The microelectrode of the invention has been set on top of the cells without touching them. Triton® X-100 1% or digitonin 50 µM was added in the perfusion medium to disrupt the integrity of the cellular membrane. This treatment induced an increase in oxidation current at the microelectrode which is due to the release of the intracellular stores of D-serine (Fig 4). An estimation of the intracellular stores of D-serine can be given by measuring the integrated surface of the oxidation current. This method allows therefore the measurement of the intracellular stores of D-serine of cells in culture.

### Example 6: in vivo recordings in the CNS of an anesthetized rat

Male Wistar rats weighing between 300 and 400g were anesthesized with chloral hydrate and placed in a stereotaxic apparatus. The reference electrode was laid on the surface of the skull and the working electrode was inserted in the cerebellum at about 1.5 mm under the pial surface.

After a 30 min stabilization time, the oxidation current at the working electrode under a potential of 0,5V vs. Ag/AgCl was recorded.

D-serine was then injected intraperitoneally at a concentration of 1g/kg of body weight.

After a few minutes the signal by our method was increased by about 90 pA, suggesting that D-serine penetrating through the blood-brain barrier into the central nervous system was detected by the microelectrode as defined in example 1.

### Example 7: D-serine detection in rat brain extracts

### A. Preparation of brain samples

Male Wistar rats (300-400g) were decapitated under isofurane anesthesia, and the forebrain was removed and homogenized in 5 ml of 5% trichloroacetic acid (TCA) to precipitate proteins. The homogenate was then centrifuged at 20 000 g for 10 min. TCA was extracted from the supernatant using ether, before lyophilization and storage at -20°C.

### B. High pressure liquid chromatography (HPLC) measurements

Lyophilized brain extracts were dissolved in 1 ml deionized water, and 50 µl were derivatized with 0.8 mg N-acetyl-cysteine and 0.25 mg o-phthaldialdehyde in a 0.1M borate buffer (pH 10.4). HPLC measurements were performed using a Waters Alliance instrument (Waters Corporation, Guyancourt, France) with a Waters symmetry column (4.6x250mm). The column and sample compartments were kept at 30 and 4°C respectively. Flow rate was set at 1 ml/min and run time was 25 min for all analyses. L- and D-serine were detected with an isocratic method using mobile phase A (990 ml of 0.1 M sodium acetate and 10 ml tetrahydrofurane, pH 6.2) and the column was washed using mobile phase B (500 ml of 0.1 M sodium acetate, 470 ml acetonitrile, 30 ml tetrahydrofurane, pH 6.2). Amino acids derivatives were detected using a Waters fluorescence detector (excitation 344 nm, emission 443 nm), and data were acquired using the Empower Pro software package (Waters Corporation, Guyancourt, France). Calibration of D-serine detection was performed using a 7-point standard curve.

### C. Comparison between D-serine detection using HPLC or the microelectrode of the present invention.

D-serine concentration in brain extracts was estimated by two independent methods, using HPLC or the RgDAAO microelectrode (Fig. 5). HPLC estimated D-serine concentration to be about 130 µM whereas the microelectrode yielded an estimate of 139 µM. In addition, we preincubated the brain extracts with DAAO and catalase to eliminate D-amino acids and peroxide present in the medium (Fig. 5 A and Fig 5B). HPLC measurements indicated that D-serine had completely disappeared in the brain extract, whereas the microelectrode detected only a small amperometric response that amounted to about 5% of the original response before the enzymatic treatment (Fig. 5 C).

These data indicate that the microelectrode of the present invention can selectively detect D-serine in complex biological media such as brain extracts, with no more than 5-7 % deviation from HPLC measurements.

### REFERENCES

[1] Stulik K, Amatore C, Holub K, Marecek V and Kutner W (2000) Microelectrodes. Definitions, characterization and applications (technical report). Pure Appl. Chem. 72: 1483-92.
[2] G. Molla, C. Vegezzi, M.S. Pilone, L. Pollegioni, Overexpression in Escherichia coli of a recombinant chimeric Rhodotorula gracilis D-amino acid oxidase,Prot. Express. Purif., 14 (1998) 289-294

## Claims

1. Microelectrode for measuring the concentration of a D-amino acid, said microelectrode comprising:
- at least one D-amino acid oxidase (DAAO) from a yeast or a microorganism, for oxidation of said D-amino acid into at least a compound B,
- means for optimising the detection of said compound B, said means being means for the catalysis of compound B oxidation, and
- means for reducing interferences due to the oxidation of other species than compound B, wherein the means for reducing interferences are limiting oxidation of compounds other than compound B which may be oxidised by the electrode.

2. Microelectrode according to claim 1, wherein the said D-amino-acid oxidase leads to the production of hydrogen peroxide as compound B.

3. Microelectrode according to any of claim 1 or 2, wherein the D-amino-acid oxidase (DAAO) does not need the addition of a cofactor in the medium to oxidise the D-amino-acid and/or shows an activity of at least 20 units/mg.

4. Microelectrode according to claim 1, wherein the said DAAO is selected from the group comprising *Rhodotorula gracilis DAAO (RgDAAO), Trigonopsis variabilis DAAO, V. luteoalbum DAAO, F. oxysporum DAAO* and derivatives thereof .

5. Microelectrode according to any of claims 1 to 4, wherein the D-amino acid is selected from the group comprising D-alanine, D-valine, D-leucine, D-isoleucine, D-methionine, D-proline, D-phenylalanine, D-tryptophan, D-serine, D-threonine, D-tyrosine, D-cysteine, D-asparagine, D-glutamine, D-lysine, D-arginine and D-histidine, , and derivatives thereof.

6. Microelectrode according to any of claims 1 to 5, wherein the D-amino acid is D-serine.

7. Microelectrode according to any of claims 1 to 6, wherein the means for oxidation of the D-amino-acid is immobilized on the electrode by:
- Covalent immobilization,
- Entrapping D-Amino-Acid Oxidase (DAAO),
- Immobilisation into the body of the electrode, and/or
- Adsorption.

8. Microelectrode according to any of claims 1 to 7, wherein the means for optimising the detection of said compound B is selected from:
- an electrochemical pre-treatment of the electrode,
- deposition of at least one metal
- deposition of an artificial system catalysing the oxidation of compound B,
- Horse Radish peroxidase (HRP),
- use of nanoparticles, and
- a combination thereof.

9. Microelectrode according claim 8, wherein
- said electrode is a carbon based electrode,
- said deposition is a electrochemical or vacuum deposition of a metal catalysing the oxidation of compound B,
- said artificial system is an artificial peroxidase system based on Prussian Blue.

10. Microelectrode according to claim 8 or 9, wherein the metal is selected from the group comprising platinum, gold, ruthenium, rhodium, palladium, iridium, osmium, iron, chromium, nickel and tungsten.

11. Microelectrode according to any one of claims 1 to 10, wherein the means for reducing interferences is done by limiting the access and/or the contact of said compounds other than compound B with the electrode.

12. Microelectrode according to any of claims 1 to 11, wherein means for reducing interferences are selected from:
- electrogenerated polymeric membranes,
- membranes deposited from solutions of charged or neutral polymers, ,
- non polymeric charged molecule layer,
- use of at least an enzyme that degrades interfering molecule(s), and
- use of electrochemical methods allowing to discriminate between different molecules.

13. Microelectrode according to any of claims 1 to 12, wherein the electrode is chosen from the group comprising:
- electrodes based on platinum, rhodium, palladium, gold, ruthenium
- electrodes based on carbon materials, graphite and glossy carbon, carbon fibres, diamond,
- electrodes covered by nanoparticles based on noble metals, carbon materials or a mixture thereof, and
- ceramic based amperometric electrodes.

14. Microelectrode according to any of claims 1 to 13, wherein the microelectrode is a carbon fibre electrode covered by a layer of ruthenium, a layer of PPD and a layer of D-Amino-Acid Oxidase (DAAO).

15. Device for detecting and/or measuring the concentration of a D-amino-acid in a medium, comprising an electrode according to any of claims 1 to 14.

16. Method for detecting and/or measuring the concentration of a D-amino-acid in a medium *in vitro* comprising the following steps:
- a working microelectrode according to any of claims 1 to 14 is placed in said medium ,
- a working potential is applied, and
- the concentration of the D-amino acid is detected and/or measured.

17. Method according claim 16, wherein the working potential is applied by cyclic voltammetry, pulsed voltammetry/amperometry or by applying a constant potential.

18. Method for making a microelectrode according to anyone of claims 1 to 14 comprising the following steps:
- treating the electrode in order to increase its sensitivity to compound B,
- the deposition of a polymer film in order to discriminate compound B from other compounds present in the medium and which may be oxidised by the electrode,
- the deposition of a film of an enzyme oxidising compound A to compound B on the electrode, and then immobilizing said enzyme.

19. Method according claim 18, wherein the electrode is treated via a metallization

## Patentansprüche

1. Mikroelektrode zur Messung der Konzentration einer D-Aminosäure, wobei die besagte Mikroelektrode Folgendes umfasst:
- mindestens eine D-Aminosäureoxidase (DAAO) aus einer Hefe oder einem Mikroorganismus zur Oxidation der besagten D-Aminosäure in mindestens eine Verbindung B,
- Mittel zur Optimierung des Nachweises der besagten Verbindung B, wobei die besagten Mittel Mittel zur Katalyse der Oxidation der Verbindung B sind, und
- Mittel zur Reduzierung der Interferenzen aufgrund der Oxidation anderer Spezies als der Verbindung B, wobei die Mittel zur Reduzierung der Interferenzen die Oxidation von Verbindungen außer der Verbindung B begrenzen, die durch die Elektrode oxidiert werden kann.

2. Mikroelektrode nach Anspruch 1, wobei die besagte D-Aminosäureoxidase zur Herstellung von Wasserstoffperoxid als Verbindung B führt.

3. Mikroelektrode nach einem der Ansprüche 1 oder 2, wobei die D-Aminosäureoxidase (DAAO) die Zugabe eines Kofaktors in das Medium nicht erfordert, um die D-Aminosäure zu oxidieren, und/oder eine Aktivität von mindestens 20 Einheiten/mg zeigt.

4. Mikroelektrode nach Anspruch 1, wobei die besagte DAAO ausgewählt ist aus der Gruppe bestehend aus *Rhodotorula gracilis DAAO (RgDAAO), Trigonopsis variabilis DAAO, V. luteoalbum DAAO, F*. *oxysporum DAAO* und Derivaten davon.

5. Mikroelektrode nach einem der Ansprüche 1 bis 4, wobei die D-Aminosäure ausgewählt ist aus der Gruppe bestehend aus D-Alanin, D-Valin, D-Leucin, D-Isoleucin, D-Methionin, D-Prolin, D-Phenylalanin, D-Tryptophan, D-Serin, D-Threonin, D-Tyrosin, D-Cystein, D-Asparagin, D-Glutamin, D-Lysin, D-Arginin und D-Histidin und Derivaten davon.

6. Mikroelektrode nach einem der Ansprüche 1 bis 5, wobei die D-Aminosäure D-Serin ist.

7. Mikroelektrode nach einem der Ansprüche 1 bis 6, wobei das Mittel zur Oxidation der D-Aminosäure auf der Elektrode immobilisiert ist durch:
- kovalente Immobilisierung,
- Einschluss der D-Aminosäureoxidase (DAAO),
- Immobilisierung in den Körper der Elektrode, und/oder
- Adsorption.

8. Mikroelektrode nach einem der Ansprüche 1 bis 7, wobei das Mittel zur Optimierung des Nachweises der besagten Verbindung B ausgewählt ist aus:
- einer elektrochemischen Vorbehandlung der Elektrode,
- der Ablagerung mindestens eines Metalls
- der Ablagerung eines künstlichen Systems, das die Oxidation der Verbindung B katalysiert,
- der Meerrettichperoxidase (HRP)
- der Verwendung von Nanopartikeln, und
- einer Kombination daraus.

9. Mikroelektrode nach Anspruch 8, wobei
- die besagte Elektrode eine auf Kohlenstoff basierende Elektrode ist,
- die besagte Ablagerung eine elektrochemische oder Vakuum-Ablagerung eines Metalls ist, das die Oxidation der Verbindung B katalysiert.
- das besagte künstliche System ein künstliches Peroxidasesystem ist, basierend auf Preußischblau.

10. Mikroelektrode nach Anspruch 8 oder 9, wobei das Metall ausgewählt ist aus der Gruppe bestehend aus Platin, Gold, Ruthenium, Rhodium, Palladium, Iridium, Osmium, Eisen, Chrom, Nickel und Wolfram.

11. Mikroelektrode nach einem der Ansprüche 1 bis 10, wobei das Mittel zur Reduzierung von Interferenzen durch die Begrenzung des Zugangs und/oder des Kontakts der Verbindungen außer der Verbindung B zur/mit der Elektrode erfolgt.

12. Mikroelektrode nach einem der Ansprüche 1 bis 11, wobei die Mittel zur Reduzierung der Interferenzen ausgewählt sind aus:
- elektrisch erzeugten polymeren Membranen,
- Membranen, abgelagert aus Lösungen von geladenen oder neutralen Polymeren,
- einer nicht polymer geladenen Molekülschicht,
- der Verwendung mindestens eines Enzyms, das ein interferierendes Molekül/ interferierende Moleküle abbaut, und
- der Verwendung von elektrochemischen Verfahren, die ermöglichen, zwischen verschiedenen Molekülen zu unterscheiden.

13. Mikroelektrode nach einem der Ansprüche 1 bis 12, wobei die Elektrode ausgewählt ist aus der Gruppe bestehend aus:
- Elektroden, basierend auf Platin, Rhodium, Palladium, Gold, Ruthenium
- Elektroden, basierend auf Kohlenstoffmaterialien, Graphit und Glanzkohle, Kohlenstofffasern, Diamant,
- Elektroden, die mit Nanopartikeln bedeckt sind, basierend auf Edelmetallen, Kohlenstoffmaterialien oder einer Mischung daraus, und
- auf Keramik basierenden amperometrischen Elektroden.

14. Mikroelektrode nach einem der Ansprüche 1 bis 13, wobei die Mikroelektrode eine Kohlenstofffaserelektrode ist, bedeckt von einer Schicht aus Ruthenium, einer Schicht aus PPD und einer Schicht aus D-Aminosäureoxidase (DAAO).

15. Vorrichtung zum Nachweis und/oder zur Messung der Konzentration einer D-Aminosäure in einem Medium, umfassend eine Elektrode nach einem der Ansprüche 1 bis 14.

16. Verfahren zum Nachweis und/oder zur Messung der Konzentration einer D-Aminosäure in einem Medium *in vitro*, umfassend die folgenden Schritte:
- eine Arbeitsmikroelektrode nach einem der Ansprüche 1 bis 14 wird in dem besagten Medium angebracht,
- ein Arbeitspotenzial wird angewendet, und
- die Konzentration der D-Aminosäure wird nachgewiesen und/oder gemessen.

17. Verfahren nach Anspruch 16, wobei das Arbeitspotenzial durch eine cyclische Voltammetrie, eine gepulste Voltammetrie/Amperometrie oder durch die Anwendung eines konstanten Potenzials angewendet wird.

18. Verfahren zur Herstellung einer Mikroelektrode nach einem der Ansprüche 1 bis 14, umfassend die folgenden Schritte:
- Behandeln der Elektrode, um ihre Empfindlichkeit gegenüber der Verbindung B zu erhöhen,
- Ablagern eines Polymerfilms, um die Verbindung B von anderen Verbindungen zu unterscheiden, die im Medium vorhanden sind, und die durch die Elektrode oxidiert werden können,
- Ablagern eines Films eines Enzyms, das die Verbindung A zur Verbindung B oxidiert, auf der Elektrode, und dann Immobilisieren des besagten Enzyms.

19. Verfahren nach Anspruch 18, wobei die Elektrode über eine Metallisierung behandelt wird.

## Revendications

1. Microélectrode pour mesurer la concentration D-acide aminé, ladite microélectrode comprenant :
- au moins une D-acide aminé oxydase (DAAO) provenant d'une levure ou d'un microorganisme, pour l'oxydation dudit D-acide aminé en au moins un composé B,
- un moyen pour optimiser la détection dudit composé B, ledit moyen étant un moyen de catalyse de l'oxydation du composé B, et
- des moyens pour réduire les interférences causées par l'oxydation d'espèces autres que le compose B, lesdits moyens pour réduire les interférences limitent l'oxydation de composés autres que le composé B qui peuvent être oxydés par l'électrode.

2. Microélectrode selon la revendication 1, dans laquelle ladite D-acide aminé oxydase (DAAO) conduit à la production de peroxyde d'hydrogène en tant que composé B.

3. Microélectrode selon la revendication 1 ou 2, dans laquelle ladite D-acide aminé oxydase (DAAO) ne nécessite pas l'addition d'un cofacteur dans le milieu pour oxyder le D-acide aminé et/ou montre une activité d'au moins 20 unités/mg.

4. Microélectrode selon la revendication 1, dans laquelle ladite DAAO est choisi dans le groupe comprenant *Rhodotorula gracilis DAAO (RgDAAO), Trigonopsis variabilis DAAO, V. luteoalbum DAAO, F. oxysporum DAAO* et leurs dérivés.

5. Microélectrode selon l'une quelconque des revendications 1 à 4, dans laquelle le D-acide aminé est choisi dans le groupe comprenant la D-alanine, la D-valine, la D-leucine, la D-isoleucine, la D-méthionine, la D-proline, la D-phénylalanine, la D-tryptophane, la D-sérine, la D-thréonine, la D-tyrosine, la D-cysteine, la D-asparagine, la D-glutamine, la D-lysine, la D-arginine et la D-histidine, et leurs dérivés.

6. Microélectrode selon l'une quelconque des revendications 1 à 5, dans laquelle le D-acide aminé est la D-sérine.

7. Microélectrode selon l'une quelconque des revendications 1 à 6, dans laquelle le moyen pour l'oxydation du D-acide aminé est immobilisé sur une électrode par :
- immobilisation covalente,
- piégeage de la D-acide aminé oxydase (DAAO),
- immobilisation à l'intérieur du corps de l'électrode, et/ou
- adsorption.

8. Microélectrode selon l'une quelconque des revendications 1 à 7, dans laquelle le moyen pour optimiser la détection dudit composé B est choisi parmi :
- un prétraitement électrochimique de l'électrode,
- le dépôt d'au moins un métal,
- le dépôt d'un système artificiel catalysant l'oxydation du composé B,
- la peroxydase de radis noir (HRP),
- l'utilisation de nanoparticules, et
- une combinaison de ceux-ci.

9. Microélectrode selon la revendication 8, dans laquelle :
- ladite électrode est une électrode à base de carbone,
- ledit dépôt est un dépôt par voie électrochimique ou un dépôt sous vide d'un métal catalysant l'oxydation du composé B,
- ledit système artificiel est un système peroxydase artificiel à base de bleu de Prusse.

10. Microélectrode selon la revendication 8 ou 9, dans laquelle le métal est choisi dans le groupe comprenant le platine, l'or, le ruthénium, le rhodium, le palladium, l'iridium, l'osmium, le fer, le chrome, le nickel et le tungstène.

11. Microélectrode selon l'une quelconque des revendications 1 à 10, dans laquelle les moyens pour réduire les interférences est mise en oeuvre en limitant l'accès et/ou le contact desdits composés autres que le composé B avec l'électrode.

12. Microélectrode selon l'une quelconque des revendications 1 à 11, dans laquelle les moyens pour réduire des interférences sont choisis parmi :
- les membranes polymériques électrogénérées,
- les membranes déposées à partir de solution de polymères chargés, ou neutres,
- une couche de molécules chargées non polymériques,
- l'utilisation d'au moins une enzyme dégradant une(des) molécule(s) interférente(s), et
- l'utilisation de méthodes électrochimiques permettant de distinguer les différentes molécules.

13. Microélectrode selon l'une quelconque des revendications 1 à 12, dans laquelle l'électrode est choisie dans le groupe comprenant :
- les électrodes à base de platine, rhodium, palladium, or, ruthénium,
- les électrodes à base de matériaux carbonés, de graphite, et de carbone brillant, de fibres de carbones, de diamant,
- les électrodes couvertes par des nanoparticules à base de métaux nobles, de matériaux carbonés ou un mélange de ceux-ci, et
- les électrodes ampérométriques à base de céramique.

14. Microélectrode selon l'une quelconque des revendications 1 à 13, dans laquelle la microélectrode est une électrode en fibre de carbone recouverte par une couche de ruthénium, une couche de PPD et une couche de D-acide aminé oxydase (DAAO).

15. Dispositif pour détecter et/ou mesurer la concentration en D-acide aminé dans un milieu, comprenant une électrode selon lune quelconque des revendications 1 à 14.

16. Procédé pour détecter et/ou mesurer la concentration en D-acide aminé dans un milieu *in vitro* comprenant les étapes suivantes :
- une microélectrode à étudier selon l'une quelconque des revendications 1 à 14 est placé dans ledit milieu,
- un potentiel de travail est appliqué, et
- la concentration du D-acide aminé est détectée et/ou mesurée.

17. Procédé selon la revendication 16, dans lequel le potentiel de travail est appliqué par un voltampérométie cyclique, voltampérométrie/ampérométrie pulsée ou par application d'un potentiel constant.

18. Procédé de fabrication d'une microélectrode selon l'une quelconque des revendications 1 à 14 comprenant les étapes suivantes :
- traitement de l'électrode de manière à augmenter sa sensibilité au composé B,
- dépôt d'un film polymère de manière à distinguer le composé B des autres composés présent dans le milieu qui peuvent être oxydés par l'électrode,
- dépôt d'un film d'enzyme oxydant le composé A en composé B sur l'électrode, et puis immobilisation dudit enzyme.

19. Procédé selon la revendication 18, dans lequel l'électrode est traitée par une métallisation.
